# EUROPEAN PATENT APPLICATION

(11) **EP 0 537 456 A1**
(43) Date of publication of application: **21.04.1993**
(21) Application number: 92114838.3
(22) Date of filing: 31.08.1992
(51) Int. Cl.: C12N 15/81, C12N 15/56

(54) **Genetic vector for multiple stable integration of DNA sequences into the genome of the yeasts kluyveromyces lactis and saccharomyces cerevisiae and plasmids containing the same**

(30) Priority: 04.09.1991 IT MI912349
(71) Applicant: SCLAVO S.p.A., I-53100 Siena (IT)
(72) Inventor: Galeotti, Cesira Luigia, 53100 Siena (IT); Gallo, Eugenia, 53100 Siena (IT); Rossolini, Gian Maria, 53100 Siena (IT); Riccio, Maria Letizia, 53100 Siena (IT); Thaller, Maria Cristina, 00136 Rome (IT)
(74) Representative: Moretti, Giorgio

(57) **Abstract**

There is described a genetic vector for stable integration, by homologous recombination, of specific DNA sequences into the genome of the yeasts Kluyveromyces lactis and Saccharomyces cerevisiae which allows high number multiple integration events to be obtained and plasmids containing the same.

## Description

### Technical field of the invention

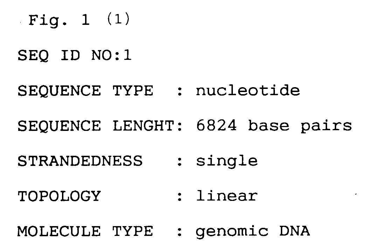

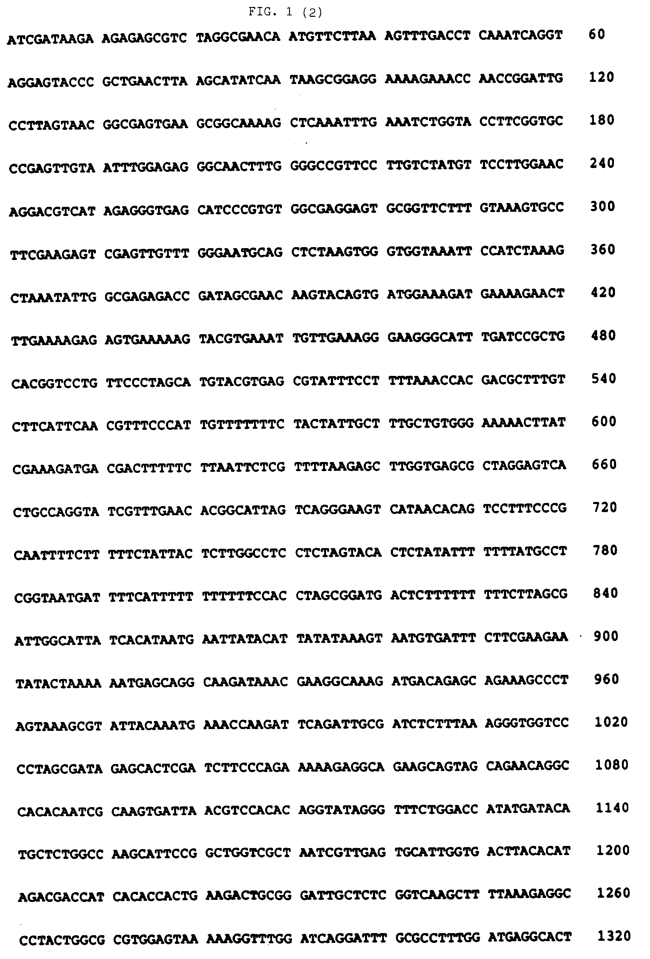

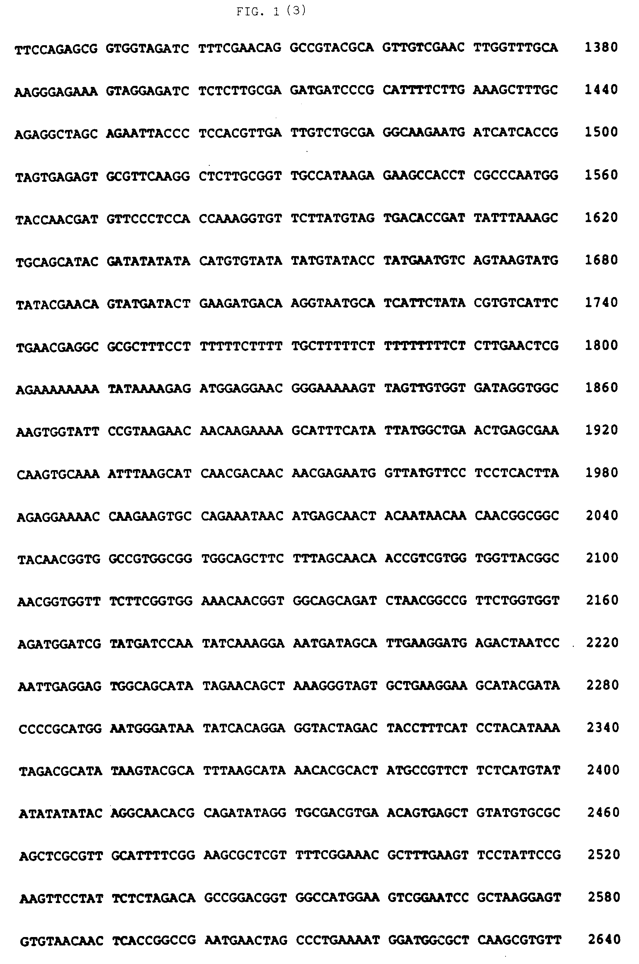

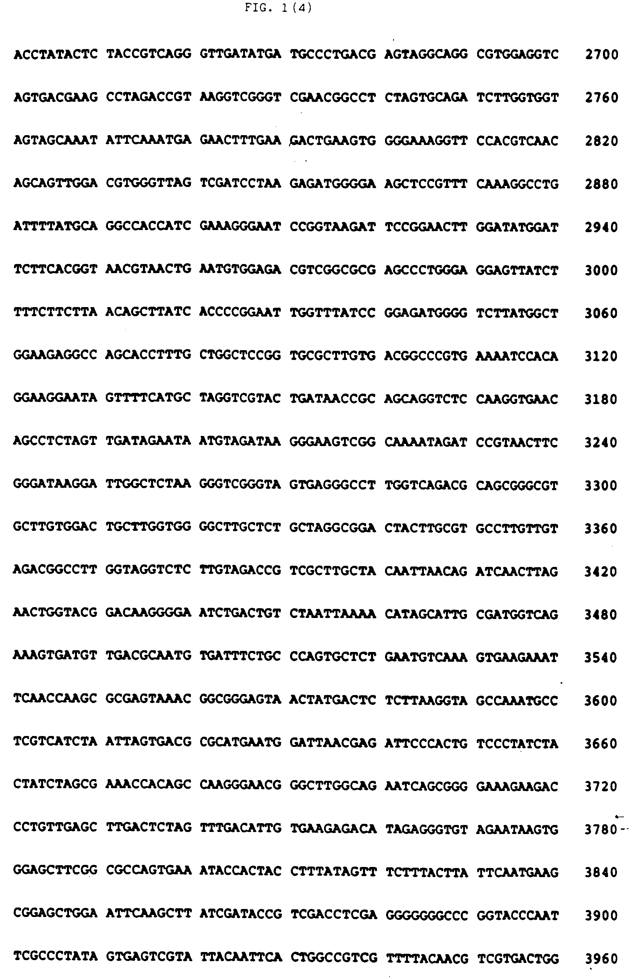

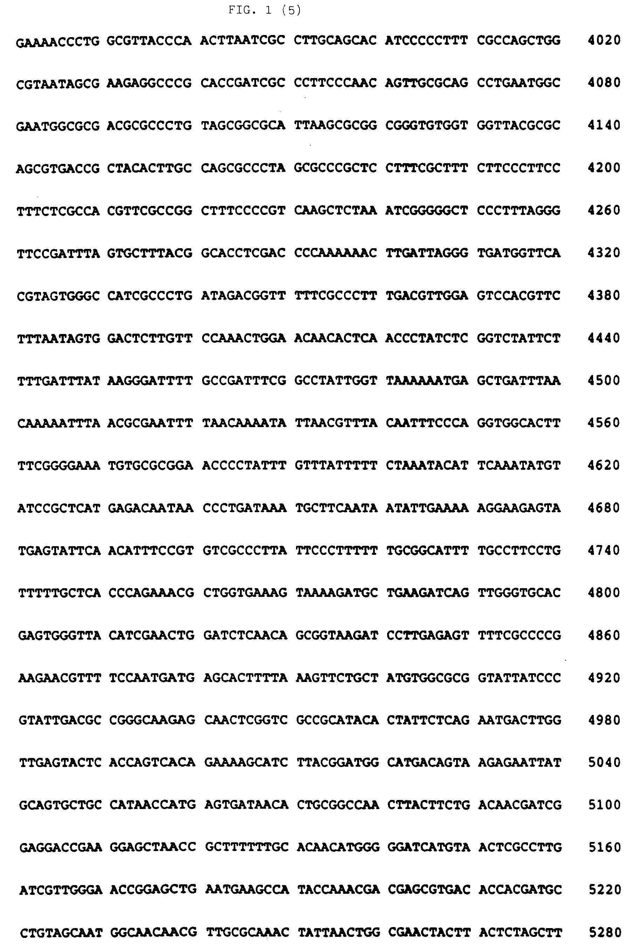

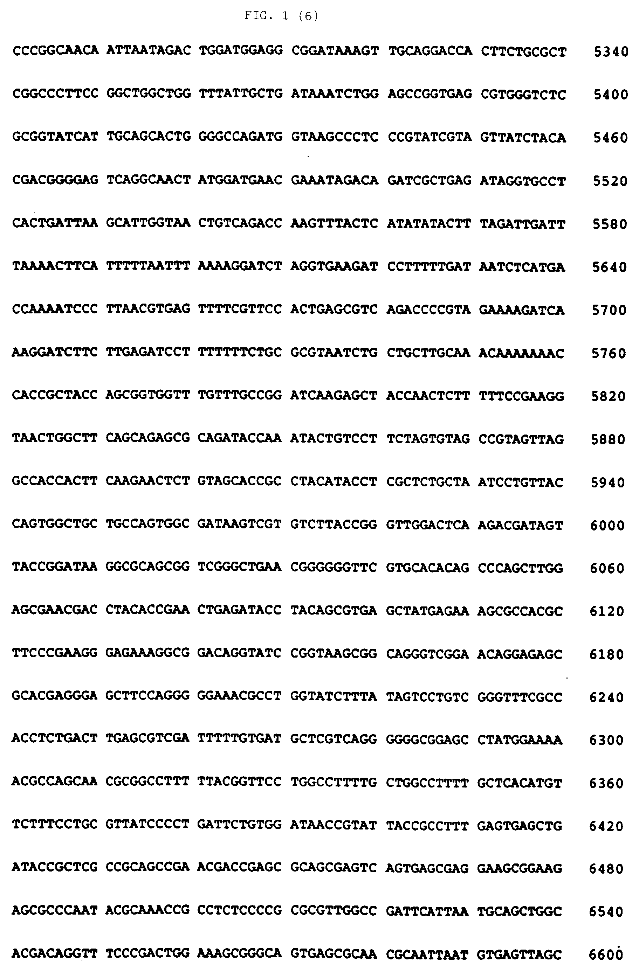

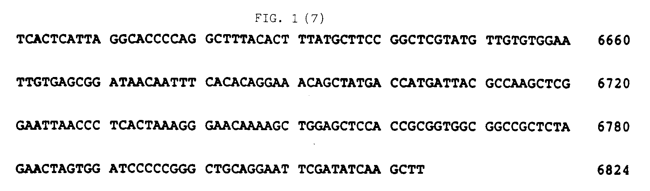

The present invention relates to a genetic vector which allows the stable multiple integration of DNA sequences into the genome of the yeasts Kluyveromyces lactis and Saccharomyces cerevisiae and consists of a sequence of 6824 bases as illustrated in Fig.1, (Sequence Listing; SEQ ID NO:1).

The invention further relates to plasmids comprising said vector.

### Background of the invention

It is known that the yeast K.lactis is already widely employed in industrial scale fermentative processes and it further represents a valid alternative to S. cerevisiae for expressing heterologous proteins of pharmaceutical interest. A few episome vectors only have been so far employed with this yeast which moreover are highly instable in non-selective growth conditions.

Moreover the taxonomic distance between K.lactis and S.cerevisiae is so important to prevent the use of vectors suitable for S.cerevisiae in K.lactis and vice-versa.

Also the integrative-type vectors cannot be equivalently employed into the two yeasts since the homology between corresponding genes rates about 70%.

### Detailed description of the invention

The present invention offers a solution to the aforementioned drawbacks in that it avails a vector which can be incorporated into the genome of both yeasts Kluyveromyces lactis and Saccharomyces cerevisiae with stable integration, in high copy number, of the expression cassette expected to be expressed by the yeast.

The vector according to the present invention is represented by the sequence of 6824 bases illustrated in Fig. 1 (Sequence Listing; SEQ ID NO: 1).

The vector according to the present invention therefore may be used both in a yeast already widely exploited for biotechnological applications (Saccharomyces cerevisiae) and a yeast, such as Kluyveromyces lactis , which represents an alternative host to the former and which is highly interesting for the above application; this property is of specific importance in view of the present paucity of genetic vectors suitable for Kluyveromyces lactis.

A further advantage of the present vector is the fact that, unlike the case with the traditional integrating vectors, multiple integration events can be easily obtained also in high copy number. The vector according to the invention is also more stable than the episome vectors available at present.

A further object of the present invention is an integrating plasmid comprising the vector of the invention.

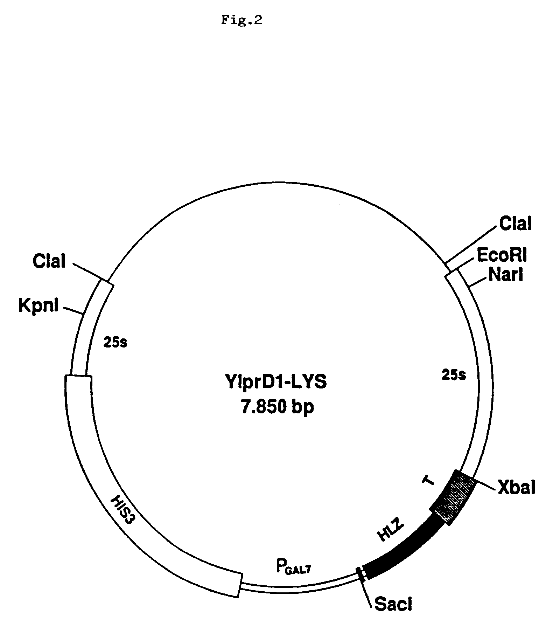

Said integrating plasmid comprises in particular a region necessary for the maintenance of the plasmid state in Escherichia coli (bacterial host in which usually the genetic manipulations are carried out before the transfer into yeast) and a domain which acts as the integrating unit consisting of two not contiguous sequences of the 255 ribosomal DNA from Saccharomyces cerevisiae, flanking a genetical marker suitable for selection of the yeast transformants in which the integration event occurred. Within the integration unit, other DNA sequences may be introduced such as, for example, expression cassettes. Once the vector has been prepared in E.coli with the desired configuration (genetic marker, expression cassettes or other DNA sequences), the integration unit is cleaved with suitable restriction enzymes and used for the transformation of the yeast. In particular it has been used the gene HIS3 from Kluyveromices lactis and Saccharomyces cerevisiae as genetic marker for the selection of the transformants and an expression cassette for the production and secretion into the culture medium of human lysozyme. By transforming with this construct HIS3⁻ strains of Kluyveromices lactis and Saccharomyces cerevisiae, transformants have been obtained in which the integration unit was integrated in more copy number (ranging from 3-4 to 40) within the yeast ribosomal DNA and which produced recombinant human lysozyme which was secreted into and recovered from the culture medium. The map of the integration plasmid according to the invention (identified by the designation YIprD1-LYS), which consists of 7850 kb, is reported in Fig. 2 and includes the aforementioned integration vector and an expression cassette comprising the Kluyveromices lactis GAL7 promoter [T.D. Webster and R.C. Dickson, Nucleic Acid Res., 16, 8192-8194 (1988)], the signal sequence of the K.lactis killer toxin (SacI), the cDNA sequence coding for the ripe form of human lysozyme (HLZ) and the transcription termination signal FLP of the plasmid 2µ from S.cerevisiae (T). In order to collocate the integration unit in the correct position of the chromosomal rDNA, the plasmid YIprD1-LYS has been cleaved with ClaI. Transformants obtained by complementing the mutation HIS3⁻ of the strain K.lactis WM37 [M. Tingle et al., Genetics 58, 361-371 (1968)] with the ClaI cleaved YIprD1-LYS DNA have been tested for the expression of heterologous proteins by growing them in YP culture medium comprising galactose as carbon source and M. luteus cells as substrate for detecting the lysozyme activity. All the obtained transformants proved to be capable of secreting lysozyme, as shown by a clear halo of M. luteus lysed cells around the colonies. In Table 1 there are reported the results obtained by quantitatively analysing the lysozyme activity in the culture medium of K.lactis trasformants grown in selective or non-selective medium comprising galactose as carbon source.

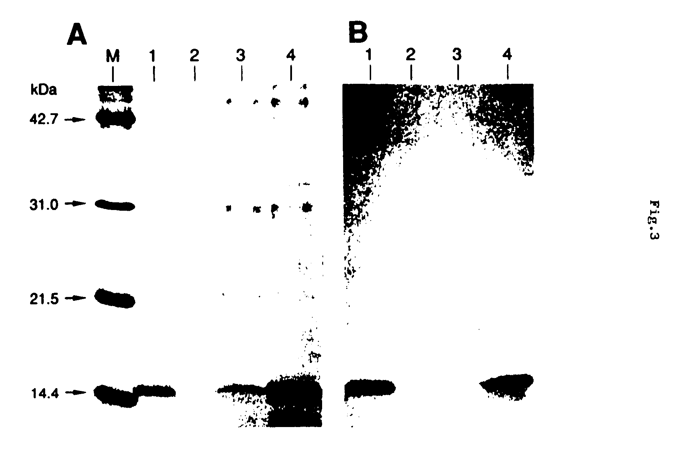

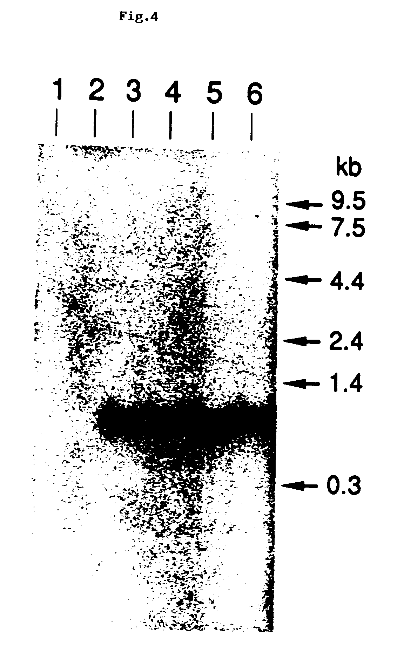

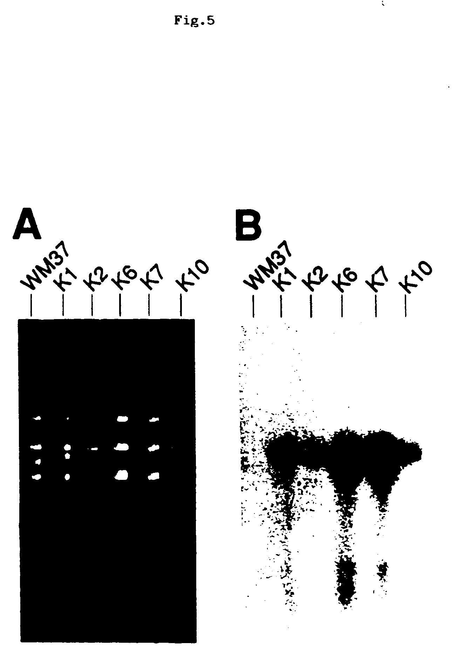

As a comparative example, there are reported, in the same Table 1, the values obtained by testing in the same way S. cerevisiae transformants harbouring the episome expression vector YEpsec1 [Baldari et al., The EMBO Journal 6, 229-234 (1987)] comprising the same human lysozyme cDNA sequence of YIprD1-LYS. As expectable from an episome expression system, all the analysed S. cerevisiae/YEpsec-LYS transformants secreted the heterologous protein in similar amount and the expression level was higher in complete medium. On the contrary, different transformants of K. lactis/YIprD1-LYS showed different amounts of secreted lysozyme and moreover the detected amount of heterologous protein was always higher upon growing in selective medium. Since the stability of the transformants may play a role for the observed difference in results among the K.lactis/YIprD1-LYS transformants, they were analysed to check their growing ability on selective medium and capability of secreting lysozyme upon a growing of more than 20 generations in non-selective medium. All the transformants exhibited a remarkable mitotic stability and proved to be still positive by the M. luteus assay. Gel electrophoresis and Western Blot analysis of the supernatant of two transformants which showed the highest production of secreted lysozyme (K6 and K7), confermed the results obtained by quantitative assays, in particular the existence of a single band of recombinant product showing the same properties of the human purified lysozyme (Fig. 3). The difference in expression at mRNA level for said two transformants, by considering for each transformant the two optional culture conditions, was evaluated by Northern Blot hybridisation. The amount of lysozyme-specific RNA present in the cells of the two considered transformants, either in selective or in non-selective medium, corresponds to the different amounts observed at protein level (Fig. 4). It may therefore be argued that the different expression level among the transformants may be due to a difference in the copy number and/or in location within the integration sites. The chromosomal integration sites of the expression cassettes integrated in YIprD1-LYS was identified in different transformants by Southern blotting of intact TAFE-separated chromosomes (Fig. 5A). As illustrated in Fig. 5B, a lysozyme-specific probe hybridized with the same chromosome in all the transformants. A hybridisation signal at the same position was also obtained by using the 25 S fragment of 2.4 Kb EcoRI as probe. This result suggests that the integration of the expression cassette into YIprD1-LYS occurred in the same position of the chromosomal DNA in all the transformants. The difference in the signal intensity among the transformants analysed with lysozyme-specific probes may be attributed to differences in the number of the integrated copies.

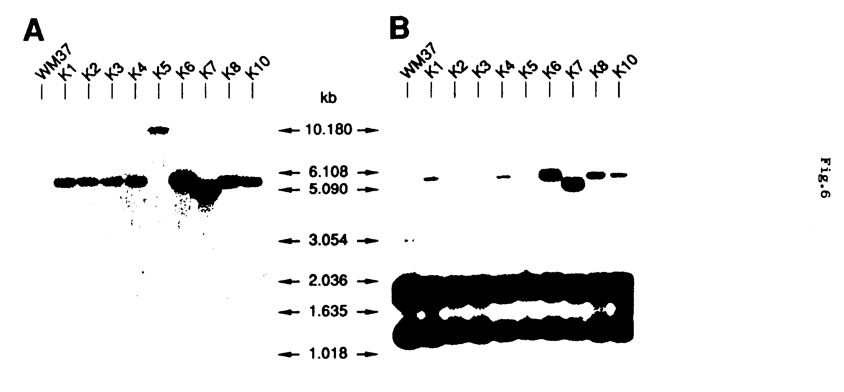

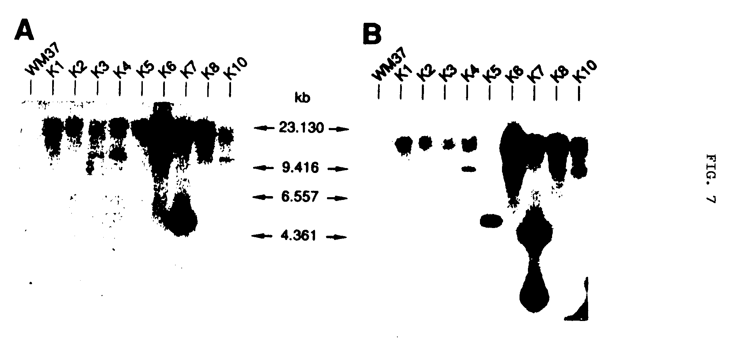

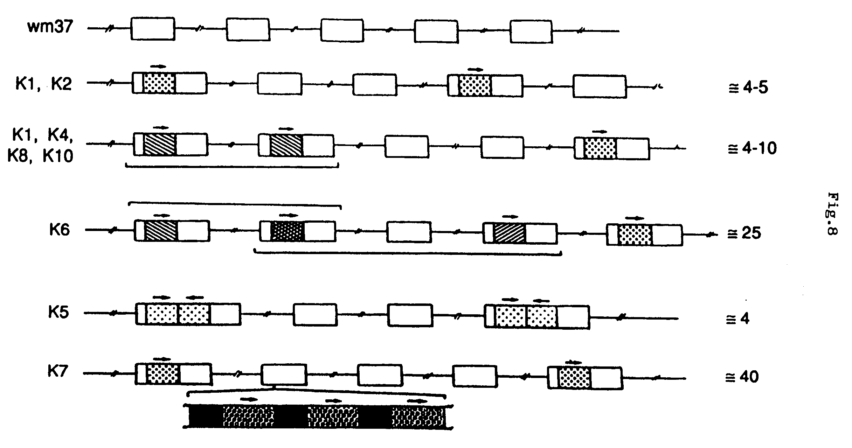

Fig. 6 shows the results of an analysis of the copy number carried out by hybridisation of the transformant total DNA after digestion with ClaI. The same samples have been hybridized with a lysozyme-specific probe (Fig. 6A) and with a 25S-specific probe (Fig.6B). In many cases a 5.5 Kb band hybridized to the lysozyme-specific probes; therefore it may be argued that single copies of the integration unit YIprD1-LYS were integrated in the same position, within a repeated rDNA unit, in different transformants. The contemporary integration of two units in head-to-head configuration may on the contrary explain the hybridisation signal observed with the transformant K5. The size of the band obtained from the DNA of the transformant K7 exactly corresponds to the size of the plasmid YIprD1-LYS, indicating that the plasmid in its entirety was integrated into the rDNA of this transformant (see Fig. 7B). Clearly the transformants of Fig. 6 differ not only because of the integration mechanism of the unit but also because of the number of the integrated copies. Fig. 6B shows the relative intensity of the hybridisation signals obtained by using as probe the 2.4 kb EcoRI fragment of the 25 S rDNA. The two inferior bands, showing signals of the same intensity for all the samples, represent the endogenous 25 S DNA digested by ClaI. The comparison of the intensity among the signals of the integrated units (higher band) for each sample and the signal corresponding to the endogenous 25 S was carried out by making use of a densitometer. The number of the integrated copies, calculated for the different transformants, rates between 4 and 40 (Fig. 8) taking 115 copies as the reference for the number of rDNA repeats [R. Maleszka and G. D. Clark-Walker, Curr. Gen. 16 429-432 (1989)]. Moreover transformants which likely bear the YIperD1-LYS unit integrated in the same position of the rDNA repeat (K1, K2, K3, K4, K6, K8 and K10) may be argued, from Fig. 6, to exhibit a fivefold-difference in the number of integrated copies. In order to ascertain the distribution of the integration sites along the rDNA of each transformant, SmaI digestion of the total DNA have been analysed by Southern hybridisation (Fig. 7), by using, as probes, lysozyme cDNA and a HIS3⁻ specific probe (plasmid pBR322-HIS3). Since the K.lactis rDNA does not comprise SmaI cleavage sites, the random distribution of the integrated units should give rise to a blot hybridisation signal. This was actually observed with all the transformants when a lysozyme-specific probe was employed. Individual bands have moreover been observed with the exception of the transformants K1 and K2. The integration in contiguous rDNA repeats would explain the presence of the 10 kb band in K3, K4, K6, K8 and K10. The transformant K6 also shows an individual band of larger size which could derive from the integration in alternated rDNA repeats. Most of the transformants exhibit the same hybridisation model with both probes (lysozyme cDNA and pBR322-HIS3); K5 and K7 differ in that K5 shows one single SmaI band with the probe HIS3 only, while K7 shows two very strong bands with the same probe (Fig. 7B). The result obtained with K5 indicates that the ClaI fragment of YIprD1-LYS is integrated with a head-to-head configuration. The size of the hybridisation signals of K7 (4.85 kb and 2.9 kb) correlates with the size obtained after SmaI digestion of the DNA of the YIprD1-LYS plasmid. This confirms what was already observed upon ClaI digestion of K7 total DNA (Fig. 6), namely that multiple copies of plasmid DNA in its entirety have been integrated within one single site. Similar conclusions have been derived after the Southern Blot analysis of the total DNAs from different transformants digested with BamHI or PstI. A schematic construction of said analysis is illustrated in Fig. 8.

**TABLE 1**

| Human lysozyme secreted by transformant yeasts | | | |
|---|---|---|---|
| yeast | medium | | |
| | SDGAL (g/ml) | | YPGAL (g/ml) |
| S. cerevisiae/YEpsec-LYS | 1. | n.d. | 2.5 |
| | 2. | 0.5 | 2.5 |
| | 3. | n.d. | 2.0 |
| | 4. | 0.4 | 2.5 |
| K.lactis WM37/YIperD1-LYS | K1 | 0.85 | 0.1 |
| | K4 | 1.0 | 0.4 |
| | K5 | 1.6 | 0.35 |
| | K6 | 2.45 | 0.1 |
| | K7 | 7.0 | 0.2 |
| | K8 | 1.8 | 1.0 |
| | K10 | 0.8 | 0.4 |

## Claims

1. Genetic vector represented by the following sequence (SEQ ID NO:1):

ATCGATAAGA AGAGAGCGTC TAGGCGAACA ATGTTCTTAA AGTTTGACCT CAAATCAGGT 60

AGGAGTACCC GCTGAACTTA AGCATATCAA TAAGCGGAGG AAAAGAAACC AACCGGATTG 120

CCTTAGTAAC GGCGAGTGAA GCGGCAAAAG CTCAAATTTG AAATCTGGTA CCTTCGGTGC 180

CCGAGTTGTA ATTTGGAGAG GGCAACTTTG GGGCCGTTCC TTGTCTATGT TCCTTGGAAC 240

AGGACGTCAT AGAGGGTGAG CATCCCGTGT GGCGAGGAGT GCGGTTCTTT GTAAAGTGCC 300

TTCGAAGAGT CGAGTTGTTT GGGAATGCAG CTCTAAGTGG GTGGTAAATT CCATCTAAAG 360

CTAAATATTG GCGAGAGACC GATAGCGAAC AAGTACAGTG ATGGAAAGAT GAAAAGAACT 420

TTGAAAAGAG AGTGAAAAAG TACGTGAAAT TGTTGAAAGG GAAGGGCATT TGATCCGCTG 480

CACGGTCCTG TTCCCTAGCA TGTACGTGAG CGTATTTCCT TTTAAACCAC GACGCTTTGT 540

CTTCATTCAA CGTTTCCCAT TGTTTTTTTC TACTATTGCT TTGCTGTGGG AAAAACTTAT 600

CGAAAGATGA CGACTTTTTC TTAATTCTCG TTTTAAGAGC TTGGTGAGCG CTAGGAGTCA 660

CTGCCAGGTA TCGTTTGAAC ACGGCATTAG TCAGGGAAGT CATAACACAG TCCTTTCCCG 720

CAATTTTCTT TTTCTATTAC TCTTGGCCTC CTCTAGTACA CTCTATATTT TTTTATGCCT 780

CGGTAATGAT TTTCATTTTT TTTTTTCCAC CTAGCGGATG ACTCTTTTTT TTTCTTAGCG 840

ATTGGCATTA TCACATAATG AATTATACAT TATATAAAGT AATGTGATTT CTTCGAAGAA 900

TATACTAAAA AATGAGCAGG CAAGATAAAC GAAGGCAAAG ATGACAGAGC AGAAAGCCCT 960

AGTAAAGCGT ATTACAAATG AAACCAAGAT TCAGATTGCG ATCTCTTTAA AGGGTGGTCC 1020

CCTAGCGATA GAGCACTCGA TCTTCCCAGA AAAAGAGGCA GAAGCAGTAG CAGAACAGGC 1080

CACACAATCG CAAGTGATTA ACGTCCACAC AGGTATAGGG TTTCTGGACC ATATGATACA 1140

TGCTCTGGCC AAGCATTCCG GCTGGTCGCT AATCGTTGAG TGCATTGGTG ACTTACACAT 1200

AGACGACCAT CACACCACTG AAGACTGCGG GATTGCTCTC GGTCAAGCTT TTAAAGAGGC 1260

CCTACTGGCG CGTGGAGTAA AAAGGTTTGG ATCAGGATTT GCGCCTTTGG ATGAGGCACT 1320

TTCCAGAGCG GTGGTAGATC TTTCGAACAG GCCGTACGCA GTTGTCGAAC TTGGTTTGCA 1380

AAGGGAGAAA GTAGGAGATC TCTCTTGCGA GATGATCCCG CATTTTCTTG AAAGCTTTGC 1440

AGAGGCTAGC AGAATTACCC TCCACGTTGA TTGTCTGCGA GGCAAGAATG ATCATCACCG 1500

TAGTGAGAGT GCGTTCAAGG CTCTTGCGGT TGCCATAAGA GAAGCCACCT CGCCCAATGG 1560

TACCAACGAT GTTCCCTCCA CCAAAGGTGT TCTTATGTAG TGACACCGAT TATTTAAAGC 1620

TGCAGCATAC GATATATATA CATGTGTATA TATGTATACC TATGAATGTC AGTAAGTATG 1680

TATACGAACA GTATGATACT GAAGATGACA AGGTAATGCA TCATTCTATA CGTGTCATTC 1740

TGAACGAGGC GCGCTTTCCT TTTTTCTTTT TGCTTTTTCT TTTTTTTTCT CTTGAACTCG 1800

AGAAAAAAAA TATAAAAGAG ATGGAGGAAC GGGAAAAAGT TAGTTGTGGT GATAGGTGGC 1860

AAGTGGTATT CCGTAAGAAC AACAAGAAAA GCATTTCATA TTATGGCTGA ACTGAGCGAA 1920

CAAGTGCAAA ATTTAAGCAT CAACGACAAC AACGAGAATG GTTATGTTCC TCCTCACTTA 1980

AGAGGAAAAC CAAGAAGTGC CAGAAATAAC ATGAGCAACT ACAATAACAA CAACGGCGGC 2040

TACAACGGTG GCCGTGGCGG TGGCAGCTTC TTTAGCAACA ACCGTCGTGG TGGTTACGGC 2100

AACGGTGGTT TCTTCGGTGG AAACAACGGT GGCAGCAGAT CTAACGGCCG TTCTGGTGGT 2160

AGATGGATCG TATGATCCAA TATCAAAGGA AATGATAGCA TTGAAGGATG AGACTAATCC 2220

AATTGAGGAG TGGCAGCATA TAGAACAGCT AAAGGGTAGT GCTGAAGGAA GCATACGATA 2280

CCCCGCATGG AATGGGATAA TATCACAGGA GGTACTAGAC TACCTTTCAT CCTACATAAA 2340

TAGACGCATA TAAGTACGCA TTTAAGCATA AACACGCACT ATGCCGTTCT TCTCATGTAT 2400

ATATATATAC AGGCAACACG CAGATATAGG TGCGACGTGA ACAGTGAGCT GTATGTGCGC 2460

AGCTCGCGTT GCATTTTCGG AAGCGCTCGT TTTCGGAAAC GCTTTGAAGT TCCTATTCCG 2520

AAGTTCCTAT TCTCTAGACA GCCGGACGGT GGCCATGGAA GTCGGAATCC GCTAAGGAGT 2580

GTGTAACAAC TCACCGGCCG AATGAACTAG CCCTGAAAAT GGATGGCGCT CAAGCGTGTT 2640

ACCTATACTC TACCGTCAGG GTTGATATGA TGCCCTGACG AGTAGGCAGG CGTGGAGGTC 2700

AGTGACGAAG CCTAGACCGT AAGGTCGGGT CGAACGGCCT CTAGTGCAGA TCTTGGTGGT 2760

AGTAGCAAAT ATTCAAATGA GAACTTTGAA GACTGAAGTG GGGAAAGGTT CCACGTCAAC 2820

AGCAGTTGGA CGTGGGTTAG TCGATCCTAA GAGATGGGGA AGCTCCGTTT CAAAGGCCTG 2880

ATTTTATGCA GGCCACCATC GAAAGGGAAT CCGGTAAGAT TCCGGAACTT GGATATGGAT 2940

TCTTCACGGT AACGTAACTG AATGTGGAGA CGTCGGCGCG AGCCCTGGGA GGAGTTATCT 3000

TTTCTTCTTA ACAGCTTATC ACCCCGGAAT TGGTTTATCC GGAGATGGGG TCTTATGGCT 3060

GGAAGAGGCC AGCACCTTTG CTGGCTCCGG TGCGCTTGTG ACGGCCCGTG AAAATCCACA 3120

GGAAGGAATA GTTTTCATGC TAGGTCGTAC TGATAACCGC AGCAGGTCTC CAAGGTGAAC 3180

AGCCTCTAGT TGATAGAATA ATGTAGATAA GGGAAGTCGG CAAAATAGAT CCGTAACTTC 3240

GGGATAAGGA TTGGCTCTAA GGGTCGGGTA GTGAGGGCCT TGGTCAGACG CAGCGGGCGT 3300

GCTTGTGGAC TGCTTGGTGG GGCTTGCTCT GCTAGGCGGA CTACTTGCGT GCCTTGTTGT 3360

AGACGGCCTT GGTAGGTCTC TTGTAGACCG TCGCTTGCTA CAATTAACAG ATCAACTTAG 3420

AACTGGTACG GACAAGGGGA ATCTGACTGT CTAATTAAAA CATAGCATTG CGATGGTCAG 3480

AAAGTGATGT TGACGCAATG TGATTTCTGC CCAGTGCTCT GAATGTCAAA GTGAAGAAAT 3540

TCAACCAAGC GCGAGTAAAC GGCGGGAGTA ACTATGACTC TCTTAAGGTA GCCAAATGCC 3600

TCGTCATCTA ATTAGTGACG CGCATGAATG GATTAACGAG ATTCCCACTG TCCCTATCTA 3660

CTATCTAGCG AAACCACAGC CAAGGGAACG GGCTTGGCAG AATCAGCGGG GAAAGAAGAC 3720

CCTGTTGAGC TTGACTCTAG TTTGACATTG TGAAGAGACA TAGAGGGTGT AGAATAAGTG 3780

GGAGCTTCGG CGCCAGTGAA ATACCACTAC CTTTATAGTT TCTTTACTTA TTCAATGAAG 3840

CGGAGCTGGA ATTCAAGCTT ATCGATACCG TCGACCTCGA GGGGGGGCCC GGTACCCAAT 3900

TCGCCCTATA GTGAGTCGTA TTACAATTCA CTGGCCGTCG TTTTACAACG TCGTGACTGG 3960

GAAAACCCTG GCGTTACCCA ACTTAATCGC CTTGCAGCAC ATCCCCCTTT CGCCAGCTGG 4020

CGTAATAGCG AAGAGGCCCG CACCGATCGC CCTTCCCAAC AGTTGCGCAG CCTGAATGGC 4080

GAATGGCGCG ACGCGCCCTG TAGCGGCGCA TTAAGCGCGG CGGGTGTGGT GGTTACGCGC 4140

AGCGTGACCG CTACACTTGC CAGCGCCCTA GCGCCCGCTC CTTTCGCTTT CTTCCCTTCC 4200

TTTCTCGCCA CGTTCGCCGG CTTTCCCCGT CAAGCTCTAA ATCGGGGGCT CCCTTTAGGG 4260

TTCCGATTTA GTGCTTTACG GCACCTCGAC CCCAAAAAAC TTGATTAGGG TGATGGTTCA 4320

CGTAGTGGGC CATCGCCCTG ATAGACGGTT TTTCGCCCTT TGACGTTGGA GTCCACGTTC 4380

TTTAATAGTG GACTCTTGTT CCAAACTGGA ACAACACTCA ACCCTATCTC GGTCTATTCT 4440

TTTGATTTAT AAGGGATTTT GCCGATTTCG GCCTATTGGT TAAAAAATGA GCTGATTTAA 4500

CAAAAATTTA ACGCGAATTT TAACAAAATA TTAACGTTTA CAATTTCCCA GGTGGCACTT 4560

TTCGGGGAAA TGTGCGCGGA ACCCCTATTT GTTTATTTTT CTAAATACAT TCAAATATGT 4620

ATCCGCTCAT GAGACAATAA CCCTGATAAA TGCTTCAATA ATATTGAAAA AGGAAGAGTA 4680

TGAGTATTCA ACATTTCCGT GTCGCCCTTA TTCCCTTTTT TGCGGCATTT TGCCTTCCTG 4740

TTTTTGCTCA CCCAGAAACG CTGGTGAAAG TAAAAGATGC TGAAGATCAG TTGGGTGCAC 4800

GAGTGGGTTA CATCGAACTG GATCTCAACA GCGGTAAGAT CCTTGAGAGT TTTCGCCCCG 4860

AAGAACGTTT TCCAATGATG AGCACTTTTA AAGTTCTGCT ATGTGGCGCG GTATTATCCC 4920

GTATTGACGC CGGGCAAGAG CAACTCGGTC GCCGCATACA CTATTCTCAG AATGACTTGG 4980

TTGAGTACTC ACCAGTCACA GAAAAGCATC TTACGGATGG CATGACAGTA AGAGAATTAT 5040

GCAGTGCTGC CATAACCATG AGTGATAACA CTGCGGCCAA CTTACTTCTG ACAACGATCG 5100

GAGGACCGAA GGAGCTAACC GCTTTTTTGC ACAACATGGG GGATCATGTA ACTCGCCTTG 5160

ATCGTTGGGA ACCGGAGCTG AATGAAGCCA TACCAAACGA CGAGCGTGAC ACCACGATGC 5220

CTGTAGCAAT GGCAACAACG TTGCGCAAAC TATTAACTGG CGAACTACTT ACTCTAGCTT 5280

CCCGGCAACA ATTAATAGAC TGGATGGAGG CGGATAAAGT TGCAGGACCA CTTCTGCGCT 5340

CGGCCCTTCC GGCTGGCTGG TTTATTGCTG ATAAATCTGG AGCCGGTGAG CGTGGGTCTC 5400

GCGGTATCAT TGCAGCACTG GGGCCAGATG GTAAGCCCTC CCGTATCGTA GTTATCTACA 5460

CGACGGGGAG TCAGGCAACT ATGGATGAAC GAAATAGACA GATCGCTGAG ATAGGTGCCT 5520

CACTGATTAA GCATTGGTAA CTGTCAGACC AAGTTTACTC ATATATACTT TAGATTGATT 5580

TAAAACTTCA TTTTTAATTT AAAAGGATCT AGGTGAAGAT CCTTTTTGAT AATCTCATGA 5640

CCAAAATCCC TTAACGTGAG TTTTCGTTCC ACTGAGCGTC AGACCCCGTA GAAAAGATCA 5700

AAGGATCTTC TTGAGATCCT TTTTTTCTGC GCGTAATCTG CTGCTTGCAA ACAAAAAAAC 5760

CACCGCTACC AGCGGTGGTT TGTTTGCCGG ATCAAGAGCT ACCAACTCTT TTTCCGAAGG 5820

TAACTGGCTT CAGCAGAGCG CAGATACCAA ATACTGTCCT TCTAGTGTAG CCGTAGTTAG 5880

GCCACCACTT CAAGAACTCT GTAGCACCGC CTACATACCT CGCTCTGCTA ATCCTGTTAC 5940

CAGTGGCTGC TGCCAGTGGC GATAAGTCGT GTCTTACCGG GTTGGACTCA AGACGATAGT 6000

TACCGGATAA GGCGCAGCGG TCGGGCTGAA CGGGGGGTTC GTGCACACAG CCCAGCTTGG 6060

AGCGAACGAC CTACACCGAA CTGAGATACC TACAGCGTGA GCTATGAGAA AGCGCCACGC 6120

TTCCCGAAGG GAGAAAGGCG GACAGGTATC CGGTAAGCGG CAGGGTCGGA ACAGGAGAGC 6180

GCACGAGGGA GCTTCCAGGG GGAAACGCCT GGTATCTTTA TAGTCCTGTC GGGTTTCGCC 6240

ACCTCTGACT TGAGCGTCGA TTTTTGTGAT GCTCGTCAGG GGGGCGGAGC CTATGGAAAA 6300

ACGCCAGCAA CGCGGCCTTT TTACGGTTCC TGGCCTTTTG CTGGCCTTTT GCTCACATGT 6360

TCTTTCCTGC GTTATCCCCT GATTCTGTGG ATAACCGTAT TACCGCCTTT GAGTGAGCTG 6420

ATACCGCTCG CCGCAGCCGA ACGACCGAGC GCAGCGAGTC AGTGAGCGAG GAAGCGGAAG 6480

AGCGCCCAAT ACGCAAACCG CCTCTCCCCG CGCGTTGGCC GATTCATTAA TGCAGCTGGC 6540

ACGACAGGTT TCCCGACTGG AAAGCGGGCA GTGAGCGCAA CGCAATTAAT GTGAGTTAGC 6600

TCACTCATTA GGCACCCCAG GCTTTACACT TTATGCTTCC GGCTCGTATG TTGTGTGGAA 6660

TTGTGAGCGG ATAACAATTT CACACAGGAA ACAGCTATGA CCATGATTAC GCCAAGCTCG 6720

GAATTAACCC TCACTAAAGG GAACAAAAGC TGGAGCTCCA CCGCGGTGGC GGCCGCTCTA 6780

GAACTAGTGG ATCCCCCGGG CTGCAGGAAT TCGATATCAA GCTT 6824

2. Integrating plasmid consisting of a vector according to claim 1 and of an appropriate expression cassette.

3. Plasmid according to claim 2 wherein the expression cassette comprises the K. lactis GAL 7 promoter, the signal sequence of the killer toxin from K. lactis (SacI), the cDNA sequence encoding the ripe form of the human lysozyme (HLZ) and the transcription termination signal (FLP) of the 2µ plasmin from S. cerevisiae (T).

4. Strains of the yeast Saccharomyces cerevisiae modified with the plasmid according to claim 2.

5. Strains of the yeast Kluyveromyces lactis modified with the plasmid according to claim 2.

6. Strains according to claim 4 wherein the expression cassette inserted with the plasmid is the cassette according to claim 3.

7. Strains according to claim 5 wherein the expression cassette inserted with the plasmid is the cassette according to claim 3.

8. Method for the expression of proteins in yeasts wherein a vector according to claim 1 is employed.

9. Method according to claim 8 wherein the expressed protein is human lysozyme.
